# EUROPÄISCHE PATENTSCHRIFT

(11) **EP 1 798 553 B1**
(45) Veröffentlichungstag und Bekanntmachung des Hinweises auf die Patenterteilung: **09.06.2010**
(21) Anmeldenummer: 05027707.8
(22) Anmeldetag: 17.12.2005
(51) Int. Cl.: G01N 33/28

(54) **Verfahren und Sensorvorrichtung zur Überwachung der Qualität eines Schmier- oder Hydrauliköls**
Method and sensor device for monitoring the quality of lubricating oil or hydraulic oil
Procédé et dispositif de détection destiné à la surveillance de la qualité de l'huile de graissage ou de l'huile hydraulique

(43) Veröffentlichungstag der Anmeldung: 20.06.2007
(73) Patentinhaber: ARGO-HYTOS GmbH, 76703 Kraichtal-Menzingen (DE)
(72) Erfinder: Mann, Wolfgang Dr., 76684 Östringen-Tiefenbach (DE); Meindorf, Thomas Dr.R, 76689 Karlsdorf Neuthard (DE)
(74) Vertreter: Hoeger, Stellrecht & Partner Patentanwälte

(56) Entgegenhaltungen:
- EP-A- 1 098 197
- DE-A1- 10 152 777
- DE-A1- 19 706 486
- US-A- 5 604 441
- US-A- 5 824 889
- US-A1- 2003 060 984

## Beschreibung

Die Erfindung betrifft ein Verfahren zur Überwachung der Qualität eines Schmier- oder Hydrauliköls, wobei die relative Dielektrizitätskonstante sowie die elektrische Leitfähigkeit des Öls unter Berücksichtigung der Öltemperatur bestimmt werden.

Die Überwachung der Qualität von Schmier- oder Hydraulikölen gewinnt zunehmend Bedeutung im Zusammenhang mit der Zustandsüberwachung von Anlagen und Maschinen. Es ist das Ziel, Maschinenschäden und Maschinenstillstandszeiten aufgrund von Verschleiß und gealterten oder falschen Schmier- oder Hydraulikölen zu reduzieren oder sogar ganz zu vermeiden. Maschinenausfälle sind in vielen Fällen auf gealterte, falsche oder kontaminierte Schmier- und Hydrauliköle zurückzuführen.

Zur Überwachung der Qualität von Schmier- und Hydraulikölen ist es aus der Offenlegungsschrift DE 101 52 777 A1 bekannt, die Temperatur des Öls zu bestimmen sowie dessen relative Dielektrizitätskonstante, die elektrische Leitfähigkeit und den relativen Feuchtegehalt des Öls. In der genannten Offenlegungsschrift wird vorgeschlagen, die Temperatur des Öls sowie die weiteren voranstehend genannten Messwerte zu bestimmen und mittels einer Auswerteeinrichtung die einzelnen Messwerte mit einem durch die Öltemperatur bestimmten Erwartungswert zu vergleichen. Die Erwartungswerte können fest vorgegeben sein oder aber unter Verwendung des zeitlichen Verlaufs der Temperatur des Öls berechnet und entsprechend verändert werden. Anhand der Abweichung der aktuell ermittelten Messwerte von den temperaturabhängigen Erwartungswerten kann dann die Qualität des Öles beurteilt werden.

Die Zustandsveränderung von Ölen gestaltet sich aufgrund der Vielzahl von Alterungsmechanismen sehr facettenreich. Es hat sich gezeigt, dass ein Einzelvergleich physikalischer und/oder chemischer Kenngrößen des Öles mit theoretischen Erwartungswerten in manchen Fällen nur eine unzureichende Beurtellung der Qualität des Öles ermöglicht.

Aufgabe der vorliegenden Erfindung ist es, ein Verfahren der eingangs genannten Art derart weiterzubilden, dass es auf einfache Weise eine bessere Beurteilung der Qualität des Öles ermöglicht.

Diese Aufgabe wird bei einem Verfahren der gattungsgemäßen Art erfindungsgemäß dadurch gelöst, dass in einer Sensorvorrichtung die zeitlichen Verläufe der relativen Dielektrizitätskonstanten und der elektrischen Leitfähigkeit des Öls unter Berücksichtigung Öltemperatur bestimmt und mittels einer Mustererkennung bewertet werden.

Erfindungsgemäß werden in einer Sensorvorrichtung die zeitlichen Verläufe der genannten physikalischen Kenngrößen des Öles bestimmt und bewertet, wodurch eine verbesserte Beurteilung der Qualität des Öles möglich wird. Beispielsweise kann zwischen einer unkritischen langsamen Veränderung und einer beschleunigten kritischen Veränderung einer physikalischen Kenngröße unterschieden werden, auch ein Ölwechsel kann aufgrund einer abrupten Änderung der physikalischen Kenngröße erkannt und dokumentiert werden beispielsweise zum Nachweis dafür, dass Wartungsmaßnahmen korrekt durchgeführt wurden. Die Beurteilung des Öls erfolgt hierbei zentral in einer Sensorvorrichtung. Dies ermöglicht eine Zustandsüberwachung des Öls mittels eines einzigen kompakten Gerätes, das einfach handhabbar ist.

Zur Beurteilung der zeitlichen Verläufe der gemessenen physikalischen Kenngrößen kommt eine Mustererkennung zum Einsatz, beispielsweise auf Basis einer Fuzzy-Logic oder auch auf Basis eines neuronalen Netzwerkes.

Von besonderem Vorteil ist es, wenn zusätzlich zum zeitlichen Verlauf der gemessenen physikalischen Kenngrößen auch deren einzelne Messwerte, die zu einem gewissen Zeitpunkt ermittelt wurden, miteinander in Beziehung gesetzt werden zur Bewertung der Qualität des Öles.

Es kann vorgesehen sein, dass in zeitlichen Abständen selbsttätig die Temperatur des Öls sowie die relative Dielektrizitätskonstante und die elektrische Leitfähigkeit des Öls gemessen und die Messwerte zusammen mit dem Zeitpunkt der Messung in einem Speicherglied abgespeichert werden. Dies ermöglicht es, die vorzugsweise in regelmäßigen zeitlichen Abständen ermittelten Messwerte, die insbesondere gleichzeitig erfasst werden, bedarfsweise aus dem Speicherglied auszulesen, um eine Beurteilung der Qualität des Öles vorzunehmen.

Die voranstehend genannten physikalischen Kenngrößen sind von der Temperatur des Öles abhängig. Es wird deshalb auch jeweils die Öltemperatur bestimmt. Werden die Messwerte in einem Speicherglied abgespeichert, so können die Messwerte zusammen mit der Temperatur des Öles im Speicherglied hinterlegt werden, alternativ kann auch vorgesehen sein, dass die Messwerte für die Dielektrizitätskonstante und die elektrische Leitfähigkeit temperaturkompensiert abgespeichert werden. Der Temperatureinfluss auf Messfühler, die zur Bestimmung der physikalischen Kenngrößen zum Einsatz kommen, sowie auf die Messelektronik zur Auswertung der von den Messfühlern bereitgestellten Messsignalen, kann in vielen Fällen rechnerisch berücksichtigt werden. Die Temperaturabhängigkeit der gemessenen physikalischen Kenngrößen an sich ist jedoch in vielen Fällen vom jeweiligen Ölmedium abhängig. Ist das eingesetzte Ölmedium bekannt, kann der Zusammenhang zwischen Temperatur und Messsignal in einer Auswerteelektronik hinterlegt werden, so dass dann der aktuell bestimmte Messwert stets auf eine Referenztemperatur zurückgerechnet werden kann. Ist das Ölmedium nicht bekannt, so kann durch Messung bei unterschiedlichen Temperaturen die Abhängigkeit des aktuell gemessenen Messwertes von der Temperatur ermittelt werden. Die Umrechnung eines aktuell ermittelten Messwertes auf eine Referenztemperatur kann dann anhand der ermittelten Abhängigkeit erfolgen.

Von besonderem Vorteil ist es, wenn in der Sensorvorrichtung zusätzlich zur relativen Dielektrizitätskonstanten und der elektrischen Leitfähigkeit auch der relative Feuchtegehalt des Öls und/oder die Viskosität und/oder die optische Transmission des Öls unter Berücksichtigung der Öltemperatur bestimmt und miteinander sowie mit der relativen Dielektrizitätskonstanten und der elektrischen Leitfähigkeit durch eine Mustererkennung in Beziehung gesetzt werden. Die Bestimmung dieser zusätzlichen physikalischen Kenngrößen ermöglicht eine noch bessere Beurteilung der Qualität des Schmier- oder Hydrauliköls. So können beispielsweise durch Messung der optischen Transmission disperse Phasen im Öl auf konstruktiv einfache Weise bestimmt werden, beispielsweise freies Wasser oder verunreinigende Partikel. Durch derartige disperse Phasen wird die gerichtete optische Transmission des Öls reduziert.

Besonders vorteilhaft ist es, wenn die zeitlichen Verläufe des relativen Feuchtegehalts und/oder der Viskosität und/oder der optischen Transmission des Öls unter Berücksichtigung der Öltemperatur bestimmt und miteinander sowie mit den zeitlichen Verläufen der relativen Dielektrizitätskonstanten und der elektrischen Leitfähigkeit durch eine Mustererkennung in Beziehung gesetzt werden. Dies ermöglicht eine zuverlässige Beurteilung des Ölzustandes.

Es kann vorgesehen sein, dass man zusätzlich zu den voranstehend genannten physikalischen Eigenschaften auch die Partikelgrößenverteilung von Verunreinigungen des Öls bestimmt.

Insbesondere kann vorgesehen sein, dass man den zeitlichen Verlauf der Partikelgrößenvertellung von Verunreinigungen des Öls bestimmt und mit den zeitlichen Verläufen zumindest einiger der voranstehend genannten physikallschen Eigenschaften durch eine Mustererkennung in Beziehung setzt.

Eine Sensorvorrichtung, die insbesondere zur Durchführung des voranstehend genannten Verfahrens geeignet ist und die eine kompakte Bauform aufweist, kostengünstig herstellbar ist und eine verbesserte Beurteilung der Qualität des Öles ermöglicht, zeichnet sich insbesondere dadurch aus, dass sämtliche Messfühler zusammen mit der Auswerteelektronik in einem Gehäuse angeordnet sind, das einen in das zu überwachende Öl eintauchbaren und über mindestens eine Durchlassöffnung mit dem Öl in Fluidverbindung bringbaren Gehäuseabschnitt umfasst.

Die Messfühler der Sensorvorrichtungsind mit einer gemeinsamen Auswerteelektronik verbunden. Dies hat den Vorteil, dass nicht jedem Messfühler eine separate Auswerteschaltung zugeordnet werden muss. Es kann beispielsweise vorgesehen sein, dass die Auswerteelektronik im Multiplexbetrieb mit den einzelnen Messfühlern der Sensorvorrichtung gekoppelt ist. Kommen beispielsweise kapazitive Messfühler zum Einsatz, insbesondere kapazitive Messfühler zur Bestimmung der relativen Dielektrizitätskonstante und/oder des relativen Feuchtegehaltes, so können die Messfühler im Multiplexbetrieb mit einer einzigen Messschaltung der Auswerteelektronik verbunden sein, wobei der Messschaltung Signalspannungen zugeführt werden und diese aus den Signalspannungen einen Kapazitätswert ermittelt, aus dem wiederum die relative Dielektrizitätskonstante bzw. der relative Feuchtegehalt des Öles rechnerisch ermittelt werden kann. Hierdurch ergibt sich zum einen eine Verringerung der Herstellungskosten der Sensorvorrichtung, zum anderen erfordert die Sensorvorrichtung nur einen geringen Bauraum, da sie sehr kompakt ausgestaltet sein kann.

Die Sensorvorrichtung zeichnet sich auch durch eine einfache Handhabung aus, da sämtliche Messfühler in einem einzigen Gehäuse untergebracht sind, das mit einem Gehäuseabschnitt in das zu untersuchende Öl eingetaucht werden kann, wobei das Öl über mindestens eine Durchlassöffnung in das Gehäuseinnere eindringen und mit den Messfühlern in unmittelbaren Kontakt gelangen kann.

Die kombinierte Messung der relativen Dielektrizitätskonstanten und der elektrischen Leitfähigkeit des Öls ermöglicht eine zuverlässige Zustandsbewertung und lässt sich durch eine konstruktiv einfache und kostengünstige Messwerterfassung erzielen.

Von besonderem Vorteil ist es, wenn das Gehäuse zusätzliche Messfühler zur Bestimmung des relativen Feuchtegehalts und/oder der Viskosität und/oder der optischen Transmission des Öles aufnimmt, die mit der Auswerteelektronik verbunden sind. In dem Gehäuseabschnitt, der in das zu untersuchende Öl eintauchbar ist, können somit Messfühler für mehrere physikalische Kenngrößen des Öles positioniert sein, deren aktuelle Messwerte mittels einer Auswerteelektronik ausgewertet und miteinander in Beziehung gesetzt werden können. Außerdem ermöglicht die Sensorvorrichtung die Erfassung des zeitlichen Verlaufes der genannten physikalischen Kenngrößen des Öles, so dass eine zuverlässige Beurteilung der Ölqualität durchgeführt werden kann.

Bei einer besonders bevorzugten Ausführungsform weist die Sensorvorrichtung eine Trägeplatine auf, die einen Messabschnitt umfasst, der in dem in das Öl eintauchenden Gehäuseabschnitt positioniert ist und an dem vorzugsweise alle zum Einsatz kommenden Messfühler angeordnet sind. Messfühler der Sensorvorrichtung sind somit auf einer gemeinsamen Trägerplatine festgelegt, die mit einem Messabschnitt in das Öl eingetaucht werden kann, so dass die Messfühler das Öl unmittelbar kontaktieren können.

Die Trägerplatine umfasst vorzugsweise auch einen Auswerteabschnitt, der aus dem in das Öl eintauchenden Gehäuseabschnitt herausragt und an dem die Auswerteelektronik angeordnet ist. Die Trägerplatine dient somit nicht nur zur Festlegung von Messfühlern, sondern sie nimmt auch die Auswerteelektronik auf. Dies ermöglicht eine besonders kompakte Ausgestaltung der Sensorvorrichtung. Außerdem kann die Auswerteelektronik über Leiterbahnen, die in Form dünner Schichten auf die Trägerplatine aufgebracht sind, auf konstruktiv einfache Weise mit einzelnen Messfühlern elektrisch verbunden werden.

Die Auswerteelektronik umfasst bevorzugt einen Mikrocontroller, zumindest eine Messschaltung und mindestens ein Speicherglied. Die Messsignale der Messfühler können der mindestens einen Messschaltung zugeführt werden, die eine Auswertung der Messsignale vornimmt in Kooperation mit dem Mikrocontroller. Die ermittelten physikalischen Kenngrößen können dann in dem mindestens einen Speicherglied zeitabhängig und vorzugsweise auch temperaturkompensiert abgespeichert werden.

Zur Bestimmung der relativen Dielektizitätskonstanten kann ein Messfühler als Kondensator ausgestaltet sein mit zwei seitlich nebeneinander angeordneten und ineinandergreifenden Kammelektroden. An die beiden Kammelektroden kann eine Wechselspannung angelegt werden, die vorzugsweise eine Frequenz kleiner 1 MHz aufweist. Die Bestimmung der relativen Dielektrizitätskonstanten kann dadurch kapazitiv mit Hilfe eines C/U-Wandlers erfolgen.

Besonders vorteilhaft ist es, wenn die Kammelektroden an der Trägerplatine festgelegt und gegenüber dem Öl elektrisch isoliert sind. Hierzu kann eine auf die Kammelektroden abgeschiedene Schutzschicht zum Einsatz kommen, beispielsweise eine Aluminiumoxid-Schicht.

Bei einer bevorzugten Ausgestaltung der Sensorvorrichtung ist ein Messfühler zur Bestimmung der elektrischen Leitfähigkeit als Kondensator ausgeschaltet mit zwei seitlich nebeneinander angeordneten und ineinandergreifenden Kammelektroden. Sowohl die Bestimmung der elektrischen Leitfähigkeit als auch die Bestimmung der relativen Dielektizitätskonstanten kann somit mittels inelnandergreifender Kammelektroden erfolgen, die beispielsweise als Dünnschicht- oder Dickschichtelektroden ausgestaltet sein können. Die Herstellungskosten für die Sensorvorrichtung können dadurch besonders gering gehalten werden.

Im Gegensatz zu den für die Bestimmung der relativen Dielektizitätskonstanten zum Einsatz kommenden Kammelektroden tragen die zur Bestimmung der elektrischen Leitfähigkeit zum Einsatz kommenden Kammelektroden bei einer bevorzugten Ausgestaltung keine isolierende Abdeckschicht sondern sind derart ausgestaltet, dass sie das zu überwachende Öl elektrisch kontaktieren.

Die Kammelektroden können unmittelbar auf der Trägerplatine angeordnet sein, es kann jedoch auch vorgesehen sein, dass die Kammelektroden - vorzugsweise in Dünnschichttechnologie - auf einem piezoelektrischen Substrat, beispielsweise einem Quarzsubstrat, angeordnet sind, das an der Trägerplatine festgelegt ist.

Der Einsatz eines piezoelektrischen Substrates, beispielsweise eines Quarzsubstrates, ist insbesondere dann von Vorteil, wenn die Sensorvorrichtung einen Messfühler zur Bestimmung der Viskosität des Öles umfasst, denn dieser Messfühler weist bevorzugt zwei im Abstand zueinander auf dem Quarzsubstrat angeordnete Paare von seitlich nebeneinander angeordneten und ineinandergreifenden Kammelektroden auf in Form sogenannter Interdigitalkondensatoren. Ein erstes Paar von Kammelektroden dient hierbei als Sender zur Erzeugung einer elektroakustischen Oberflächenwelle, die sich entlang der das zu untersuchende Öl kontaktierenden Oberseite des Quarzsubstrates bis zum zweiten Paar von Kammelektroden ausbreitet. Die Oberflächenwelle wird vom Öl gedämpft, und die Dämpfung der Oberflächenwelle ist ein Maß für die Viskosität des Öles. Vorzugsweise wird die Oberflächenwelle mit einer Wechselspannung mit einer Frequenz von 78 MHz angeregt. Derartige Paare von auf einem Quarzsubstrat angeordneten Kammelektroden sind auch als Interdigitalwandler bekannt und werden beispielsweise in der DE 197 06 486 B4 beschrieben.

Bei einer vorteilhaften Ausführungsform sind Kammelektroden in Dickschichttechnologie auf ein Keramiksubstrat aufgebracht.

Ein Messfühler zur Bestimmung des relativen Feuchtegehalts des Öls ist bei ' einer bevorzugten Ausgestaltung der Sensorvorrichtung als Kondensator ausgestaltet, der zwei Elektroden aufweist, die zwischen sich eine Feuchtigkeit absorbierende Schicht, insbesondere eine hygroskopische Polymerschicht aufnehmen. Durch die Anlagerung von Feuchtigkeit aus dem zu überwachenden Öl wird die Kapazität des Messfühlers verändert. Diese Kapazität kann in üblicher Weise durch Anlegen einer Wechselspannung ermittelt werden.

Vorzugsweise ist eine erste Elektrode des Messfühlers zur Bestimmung des relativen Feuchtegehalts als leitende Metallschicht unmittelbar auf die Trägerplatine aufgebracht, und auf dieser ersten Elektrode ist die hygroskopische Polymerschicht angeordnet, die wiederum durch eine weitere, permeable Metallschicht abgedeckt ist.

Zur Bestimmung der optischen Transmission des Öles kommt bevorzugt ein Messfühler mit zwei Lichtleitern zum Einsatz, die mit einer Lichtquelle bzw. einem Lichtdetektor verbunden sind und deren freie Enden in dem in das zu überwachende Öl eintauchbaren Gehäuseabschnitt einander zugewandt sind. Als Lichtquelle kommt bevorzugt eine Leuchtdiode zum Einsatz, und der Lichtdetektor ist bevorzugt als Fotodiode ausgebildet. Insbesondere durch die Einlagerung disperser Phasen in das zu untersuchende Öl kann dessen Transmission beeinträchtigt werden, wobei als disperse Phasen an erster Stelle eingelagertes Wasser und verunreinigende Partikel zu nennen sind.

Von besonderem Vorteil ist es, wenn das Gehäuse der Sensorvorrichtung einen Verbindungsabschnitt aufweist zum Herstellen einer lösbaren Verbindung mit einem Öl aufnehmenden Hydraulikteil. Hierzu kann das Gehäuse beispielsweise einen Gewindeabschnitt aufweisen, der sich vorzugsweise an den in das Öl eintauchbaren Gehäuseabschnitt anschließt. Die Sensorvorrichtung kann dadurch unmittelbar in ein Hydraulikteil eingeschraubt werden, das das zu untersuchende Öl aufnimmt. Bei dem Hydraulikteil kann es sich beispielsweise um eine Hydraulikleitung, eine Filtervorrichtung oder um einen Vorratsbehälter für Öl handeln.

Die nachfolgende Beschreibung einer bevorzugten Ausführungsform der Erfindung dient im Zusammenhang mit der Zeichnung der näheren Erläuterung. Es zeigen:
- Figur 1:: eine schematische Schnittdarstellung einer Sensorvorrichtung;
- Figur 2:: ein Blockschaltbild der Sensorvorrichtung aus Figur 1;
- Figur 3:: eine schematische Darstellung eines Messfühlers zur Bestimmung des relativen Feuchtegehalts eines Öls;
- Figur 4:: eine schematische Darstellung eines Messfühlers zur Bestimmung der relativen Dielektrizitätskonstanten des Öles und
- Figur 5:: eine schematische Darstellung eines Messfühlers zur Bestimmung der Viskosität des Öles.

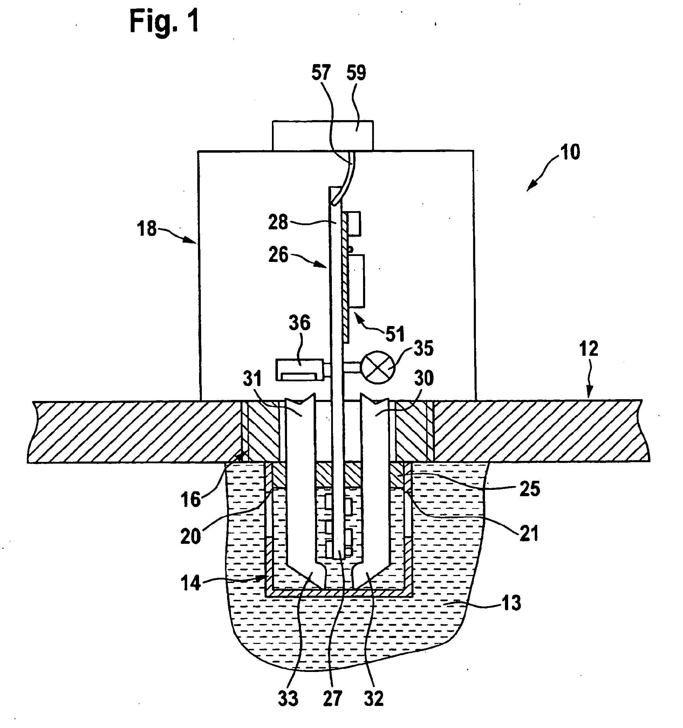

In Figur 1 ist schematisch eine Sensorvorrichtung 10 dargestellt zur Überwachung der Qualität eines Schmier- oder Hydraullköls 13 in einem Behälter 12. Diese Sensorvorrichtung 10 umfasst einen in den Behälter 12 und damit in das Öl 13 eintauchenden unteren Gehäuseabschnitt 14, der über einen Gewindeabschnitt 16 mit einem aus dem Behälter 12 herausragenden oberen Gehäuseabschnitt 18 verbunden ist.

Der untere Gehäuseabschnitt 14 ist zylindersymmetrisch ausgebildet und weist zwei einander diametral gegenüberliegende Durchgangsöffnungen 20, 21 auf, über die Öl in das Innere des unteren Gehäuseabschnittes 14 Öl 13 eindringen kann. Der Innenraum des unteren Gehäuseabschnittes 14 ist mittels einer Halteplatte 25 flüssigkeitsdicht vom Gewindeabschnitt 16 abgetrennt. An der Halteplatte 25 ist eine diese durchgreifende Trägerplatine 26 festgelegt, die mit einem Messabschnitt 27 in den Innenraum des unteren Gehäuseabschnittes 14 eintaucht, wohingegen ein Auswerteabschnitt 28 der Trägerplatine 26 innerhalb des oberen Gehäuseabschnittes 18 angeordnet ist.

Zusätzlich zur Trägerplatine 26 wird die Halteplatte 25 von zwei Lichtleitern 30, 31 durchgriffen, die mit ihren freien Enden 32 bzw. 33 im Innenraum des unteren Gehäuseabschnittes 14 einander zugewandt und im Abstand zueinander angeordnet sind. Im oberen Gehäuseabschnitt 18 sind eine Leuchtdiode 35 und eine Fotodiode 36 angeordnet, die jeweils an einer Seite der Trägerplatine 26 festgelegt sind. Ausgehend von der Leuchtdiode 35 kann über den Lichtleiter 30 Licht an dessen freies Ende 32 übertragen werden, das anschließend den mit Öl gefüllten Zwischenraum zwischen den freien Enden 32 und 33 durchläuft und auf den Lichtleiter 31 trifft, von dem aus das Licht zur Fotodiode 36 geleitet wird.

Die Leuchtdiode 35 stellt in Kombination mit der Fotodiode 36 und den beiden Lichtleitern 30, 31 einen Messfühler 38 zur Bestimmung der optischen Transmission des Öles 13 dar.

Im Messabschnitt 27 trägt die Trägerplatine 26 weitere Messfühler zur Bestimmung physikalischer Kenngrößen des Öles 13, und zwar einen Temperaturmessfühler 41 in Form eines üblichen Thermoelementes, einen Messfühler 43 zur Bestimmung der relativen Dielektrizitätskonstanten des Öles 13, einen Messfühler 45 zur Bestimmung der elektrischen Leitfähigkeit des Öles 13, einen Messfühler 47 zur Bestimmung des relativen Feuchtegehalts des Öles 13 sowie einen Messfühler 49 zur Bestimmung des Viskosität des Öles 13.

Im Bereich des Auswerteabschnittes 28 trägt die Trägerplatine 26 eine Auswerteelektronik 51, die über an sich bekannte und deshalb in der Zeichnung nicht dargestellte Leiterbahnen in Form dünner Schichten, die auf die Trägerplatine 26 aufgebracht sind, mit den voranstehend genannten Messfühlern in elektrischer Verbindung steht.

Die Auswerteelektronik 51 ist über ein mehradriges Verbindungskabel 57 mit einer außenseitig am oberen Gehäuseabschnitt 18 angeordneten Anschlussbuchse 59 verbunden, an die in üblicher Weise ein Anschlussstecker angeschlossen werden kann. Die Anschlussbuchse 59 gibt die Möglichkeit, Ausgangssignale der Auswerteelektronik 51 abzugreifen. Außerdem kann über die Anschlussbuchse 59 ein Partikelsensor 61 an die Auswerteelektronik 51 angeschlossen werden, mit dessen Hilfe eine Größenverteilung verunreinigender Partikel des Öls 13 erfasst werden kann.

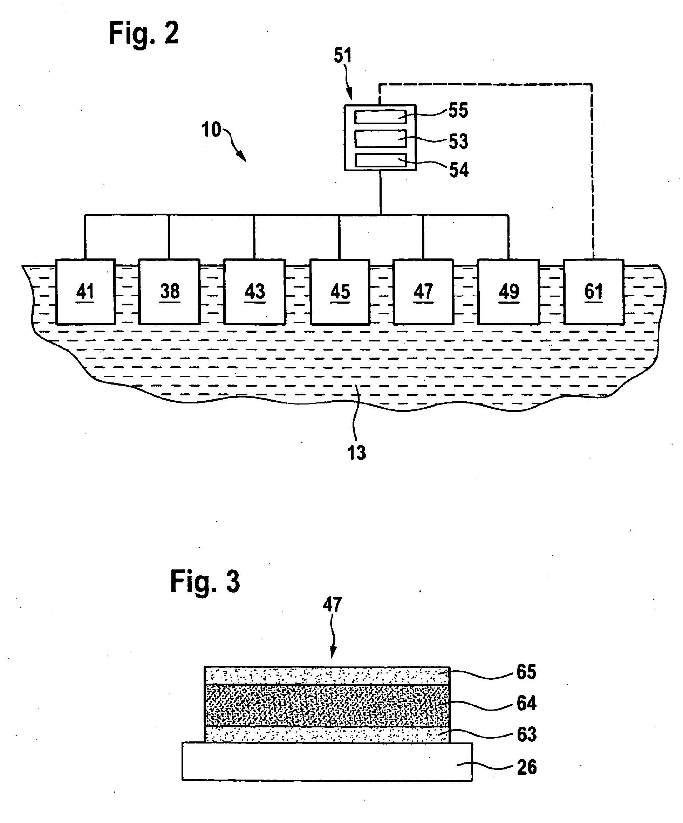

In Figur 2 ist die Sensorvorrichtung 10 nach Art eines Blockschaltbildes dargestellt. Es wird deutlich, dass sämtliche Messfühler 38, 41, 43, 45, 47, 49 sowie der Partikelsensor 61 an die Auswerteelektronik 51 angeschlossen sind, die aufgrund der zugeführten Messsignale eine Beurteilung der Qualität des Öles 13 vornimmt. Dies wird nachfolgend noch näher erläutert.

In Figur 3 ist schematisch der Messfühler 47 zur Bestimmung des relativen Feuchtegehaltes des Öles 13 dargestellt. Er ist als kapazitiver Sensor ausgebildet und umfasst eine Basiselektrode 63, die in Form einer leitenden Metallschicht unmittelbar auf die Trägerplatine 26 aufgebracht ist. Oberhalb der Basiselektrode 63 ist eine feuchtigkeitabsorbierende Schicht in Form einer hygroskopischen Polymerschicht 64 angeordnet, die von einer Deckelektrode 65 in Form einer leitenden, permeablen Metallschicht abgedeckt ist. An die Basiselektrode 63 und die Deckelektrode 65 kann über an sich bekannte und deshalb in der Zeichnung nicht dargestellte Verbindungsleitungen eine hochfrequente Messspannung angeschlossen und dadurch die Kapazität des durch die Basiselektrode 63, die Polymerschicht 64 und die Deckelektrode 65 gebildeten Kondensators bestimmt werden. Die Kapazität ist abhängig vom Feuchtegehalt der Polymerschicht 64 und damit auch vom Feuchtegehalt des Öls 13.

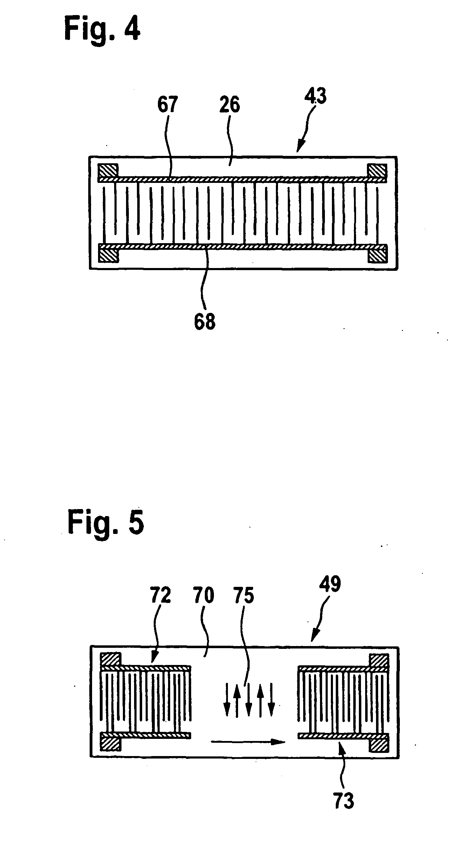

In Figur 4 ist schematisch der Messfühler 43 zur Bestimmung der relativen Dielektrizitätskonstanten des Öles 13 dargestellt. Dieser ist als Kondensator ausgebildet und weist zwei seitlich nebeneinander angeordnete und ineinandergreifende Kammelektroden 67, 68 auf, die unmittelbar auf die Trägerplatine 26 aufgebracht sind. Sie werden von einer elektrisch isolierenden Abdeckschicht überdeckt, die eine Passivierung der Kammelektroden 67, 68 sicherstellt. An die Kammelektroden 67, 68 kann über in der Zeichnung nicht dargestellte Anschlussleitungen eine hochfrequente Messspannung mit einer Frequenz kleiner 1 MHz angelegt werden, so dass in üblicher Weise die Kapazität des Kondensators bestimmt werden kann. Die Kapazität ist abhängig von der Dielektrizitätskonstanten des zwischen den beiden Kammelektroden 67, 68 angeordneten Öls 13, die somit auf einfache Weise bestimmt werden kann.

In entsprechender Weise wie der Messfühler 43 zur Bestimmung der relativen Dielektrizität des Öles 13 ist auch der Messfühler 45 zur Bestimmung der elektrischen Leitfähigkeit des Öles ausgestaltet. Er ist in der Zeichnung nicht dargestellt, weist jedoch ebenfalls zwei seitlich nebeneinander angeordnete und ineinandergreifende Kammelektroden auf, wobei diese allerdings im Unterschied zu den Kammelektroden 67, 68 das Öl 13 elektrisch kontaktieren zur Bestimmung der elektrischen Leitfähigkeit des Öles 13.

In Figur 5 ist schematisch der Messfühler 49 zur Bestimmung der Viskosität des Öles 13 dargestellt. Dieser umfasst ein piezoelektrisches Substrat in Form eines Quarzsubstrates 70, auf das ein erstes Kammelektrodenpaar 72 und im Abstand zu diesem ein zweites Kammelektrodenpaar 73 als dünne Schichten aufgebracht sind. Beide Kammelektrodenpaare 72, 73 weisen jeweils zwei seitlich nebeneinander angeordnete und ineinandergreifende Kammelektroden auf. Die Kammelektrodenpaare 72, 73 bestehen aus Gold und bilden einen Sender bzw. einen Empfänger für eine elektroakustische Oberflächenwelle 75, die sich bei Anregung des Kammelektrodenpaares 72 von diesem über die vom Öl 13 kontaktierte Oberfläche des Quarzsubstrates 70 zum zweiten Kammelektrodenpaar 73 ausbreitet. An das erste Kammelektrodenpaar 72 wird eine hochfrequente Messspannung mit einer Frequenz von 78 MHz angelegt, und von der elektroakustischen Oberflächenwelle wird im zweiten Kammelektrodenpaar 73 aufgrund der piezoelektrischen Eigenschaft des Quarzsubstrates 70 eine entsprechende Signalspannung angeregt, die mittels in der Zeichnung nicht dargestellter Verbindungsleitungen abgegriffen und von der Auswerteelektronik 51 ausgewertet werden kann. Die abgegriffene Signalspannung ist abhängig von der Dämpfung der elektroakustischen Oberflächenwelle. Die Dämpfung ist proportional zur Wurzel aus der dynamischen Viskosität multipliziert mit der Dichte des Öles 13. Die abgegriffene Signalspannung ist somit ein Maß für die relative Viskosität des Öles 13.

Sämtliche Messsignale der Messfühler 38, 41, 43, 45, 47, 49 werden der Auswerteelektronik 51 zugeführt, die in gleichbleibenden zeitlichen Abständen entsprechende Messungen der genannten physikalischen Kenngrößen des Öles 13 durchführt und die erhaltenen Messergebnisse unter Berücksichtigung der Temperatur des Öles 13 im Speicherglied 55 abspeichert. Dies ermöglicht es, die zeitlichen Verläufe der relativen Dielektrizitätskonstanten, der elektrischen Leitfähigkeit, des relativen Feuchtegehaltes sowie der optischen Transmission und der Partikelgrößenverteilung von Verunreinigungen des Öles 13 auf einfache Weise zu bestimmen und in der Auswerteelektronik 51 miteinander in Beziehung zu setzen zur Beurteilung der Qualität des Öles 13.

Es können somit mittels der Auswerteelektronik 51 sowohl die gemessenen Kenngrößen (bezogen auf eine Referenztemperatur) als auch die zeitlichen Änderungen dieser Kenngrößen mit Hilfe des Mikrocontrollers 53 bewertet werden. Der Mikrocontroller 53 kann hierzu eine Mustererkennung auf Basis einer Fuzzy-Logic oder auf Basis eines neuronalen Netzes durchführen. Dadurch kann eine zuverlässige Zustandsbewertung des Öles 13 durchgeführt werden.

Je nach Ergebnis der Beurteilung durch die Auswerteelektronik 51 kann von dieser über die Anschlussbuchse 59 ein Ausgangssignal ausgegeben werden, das beispielsweise einer Anzeigevorrichtung und/oder einer Maschinensteuerung zugeführt werden kann.

## Patentansprüche

1. Verfahren zur Überwachung der Qualität eines Schmier- oder Hydrauliköls, wobei die relative Dielektrizitätskonstante und die elektrische Leitfähigkeit des Öls unter Berücksichtigung der Öltemperatur bestimmt werden, **dadurch gekennzeichnet, dass** in einer Sensorvorrichtung (10) die zeitlichen Verläufe der relativen Dielektrizitätskonstanten und der elektrischen Leitfähigkeit des Öls unter Berücksichtigung der Öltemperatur bestimmt und mittels einer Mustererkennung bewertet werden.

2. Verfahren nach Anspruch 1, **dadurch gekennzeichnet, dass** in zeitlichen Abständen selbsttätig die Temperatur des Öls sowie die relative Dielektrizitätskonstante und die elektrische Leitfähigkeit des Öls gemessen und die Messwerte zusammen mit dem Zeitpunkt der Messung in einem Speicherglied abgespeichert werden.

3. Verfahren nach Anspruch 2, **dadurch gekennzeichnet, dass** die Messwerte für die relative Dielektrizitätskonstante und die elektrische Leitfähigkeit des Öls temperaturkompensiert abgespeichert werden.

4. Verfahren nach einem der voranstehenden Ansprüche, **dadurch gekennzeichnet, dass** in der Sensorvorrichtung (10) zusätzlich der relative Feuchtegehalt und/oder die Viskosität und/oder die optische Transmission des Öls unter Berücksichtigung der Öltemperatur bestimmt und miteinander sowie mit der relativen Dielektrizitätskonstanten und der elektrischen Leitfähigkeit durch eine Mustererkennung in Beziehung gesetzt werden.

5. Verfahren nach Anspruch 4, **dadurch gekennzeichnet, dass** die zeitlichen Verläufe des relativen Feuchtegehalts und/oder der Viskosität und/oder der optischen Transmission des Öls unter Berücksichtigung der Öltemperatur bestimmt und miteinander sowie mit den zeitlichen Verläufen der relativen Dielektrizitätskonstanten und der elektrischen Leitfähigkeit durch eine Mustererkennung in Beziehung gesetzt werden.

6. Verfahren nach einem der voranstehenden Ansprüche, **dadurch gekennzeichnet, dass** die Partikelgrößenverteilung von Verunreinigungen des Öls bestimmt wird.

7. Verfahren nach Anspruch 6, **dadurch gekennzeichnet, dass** man den zeitlichen Verlauf der Partikelgrößenverteilung von Verunreinigungen des Öls bestimmt und mit den zeitlichen Verläufen der relativen Dielektrizitätskonstanten und der elektrischen Leitfähigkeit des Öls durch eine Mustererkennung in Beziehung setzt.

## Claims

1. Method for monitoring the quality of a lubricating oil or hydraulic oil, wherein the relative dielectric constant and the electric conductivity of the oil are determined taking into account the oil temperature, **characterized in that** in a sensor device (10) the time-dependent courses of the relative dielectric constant and the electric conductivity of the oil are determined taking into account the oil temperature and are evaluated by means of a pattern recognition.

2. Method in accordance with claim 1, **characterized in that** at intervals of time the temperature of the oil and the relative dielectric constant and the electric conductivity of the oil are automatically measured, and the measured values are stored together with the time of measurement in a storage element.

3. Method in accordance with claim 2, **characterized in that** the measured values for the relative dielectric constant and the electric conductivity of the oil are stored with temperature compensation.

4. Method in accordance with any one of the preceding claims, **characterized in that** in the sensor device (10) the relative moisture content and/or the viscosity and/or the optical transmission of the oil are additionally determined taking into account the oil temperature and are related to one another and to the relative dielectric constant and the electric conductivity by a pattern recognition.

5. Method in accordance with claim 4, **characterized in that** the time-dependent courses of the relative moisture content and/or the viscosity and/or the optical transmission of the oil are determined taking into account the oil temperature and are related to one another and to the time-dependent courses of the relative dielectric constant and the electric conductivity by a pattern recognition.

6. Method in accordance with any one of the preceding claims, **characterized in that** the particle size distribution of impurities in the oil is determined.

7. Method in accordance with claim 6, **characterized in that** the time-dependent course of the particle size distribution of impurities in the oil is determined and related to the time-dependent courses of the relative dielectric constant and the electric conductivity of the oil by a pattern recognition.

## Revendications

1. Procédé de surveillance de la qualité d'une huile hydraulique ou de lubrification, dans lequel la constante diélectrique relative et la conductibilité électrique de l'huile sont déterminées en tenant compte de la température de l'huile, **caractérisé en ce que**, dans un dispositif formant capteur (10), les variations dans le temps de la constante diélectrique relative et de la conductibilité électrique de l'huile sont déterminées en tenant compte de la température de l'huile et sont évaluées par reconnaissance de formes.

2. Procédé selon la revendication 1, **caractérisé en ce que**, par intervalles de temps, on mesure automatiquement la température de l'huile ainsi que la constante diélectrique relative et la conductibilité électrique de l'huile, et on mémorise les valeurs de mesure, conjointement avec l'instant de la mesure, dans un élément de mémorisation.

3. Procédé selon la revendication 2, **caractérisé en ce que** les valeurs de mesure de la constante diélectrique relative et de la conductibilité électrique de l'huile sont mémorisées avec compensation de température.

4. Procédé selon l'une des revendications précédentes, **caractérisé en ce que**, dans le dispositif formant capteur (10), l'on détermine en plus la teneur relative en humidité et/ou la viscosité et/ou la transmission optique de l'huile en tenant compte de la température de l'huile, et l'on établit un rapport entre lesdites valeurs entre elles, ainsi qu'entre ces valeurs et la constante diélectrique relative et la conductibilité électrique par reconnaissance de formes.

5. Procédé selon la revendication 4, **caractérisé en ce que** les variations dans le temps de la teneur relative en humidité et/ou de la viscosité et/ou de la transmission optique de l'huile sont déterminées en tenant compte de la température de l'huile, et l'on établit un rapport entre lesdites variations ainsi qu'entre ces variations et les variations dans le temps de la constante diélectrique relative et de la conductibilité électrique par reconnaissance de formes.

6. Procédé selon l'une des revendications précédentes, **caractérisé en ce que** l'on détermine la distribution granulométrique des impuretés de l'huile.

7. Procédé selon la revendication 6, **caractérisé en ce que** l'on détermine la variation dans le temps de la distribution granulométrique des impuretés de l'huile, et l'on établit un rapport entre ladite variation et les variations dans le temps de la constante diélectrique relative et de la conductibilité électrique de l'huile par reconnaissance de formes.
